# EUROPÄISCHE PATENTSCHRIFT

(11) **EP 4 436 546 B1**
(45) Veröffentlichungstag und Bekanntmachung des Hinweises auf die Patenterteilung: **21.01.2026**
(21) Anmeldenummer: 22822910.0
(22) Anmeldetag: 25.11.2022
(51) Int. Cl.: A61K 9/00, A61K 9/70, A61M 5/32, A61M 25/00, A61M 37/00

(54) **APPLIKATIONSHILFE FÜR ORALEN DÜNNFILM**
APPLICATION AID FOR ORAL THIN FILM
AIDE À LA MISE EN OEUVRE POUR COUCHE MINCE ORALE

(30) Priorität: 25.11.2021 DE 102021130950
(43) Veröffentlichungstag der Anmeldung: 02.10.2024
(73) Patentinhaber: LTS Lohmann Therapie-Systeme AG, 56626 Andernach (DE)
(72) Erfinder: MÜLLER, Markus, 53840 Troisdorf (DE)
(74) Vertreter: Meissner Bolte Partnerschaft mbB
(86) Internationale Anmeldenummer: PCT/EP2022/083375
(87) Internationale Veröffentlichungsnummer: WO 2023/094638

(56) Entgegenhaltungen:
- EP-A2- 2 389 940
- WO-A1-2004/009172
- WO-A1-2007/116959
- WO-A1-2013/152092
- WO-A1-2019/088227
- WO-A1-2019/101798
- WO-A1-2019/110727
- WO-A1-2019/115815
- WO-A1-2020/169667
- WO-A1-2021/123289
- WO-A1-2022/042799
- WO-A2-02/066016
- WO-A2-2010/022326
- US-A1- 2017 080 196
- US-A1- 2018 177 991
- US-B2- 7 658 728

## Beschreibung

Die Erfindung betrifft einen Verbund, umfassend einen oralen Dünnfilm und eine Applikationshilfe für den oralen Dünnfilm, ein Verfahren zu dessen Herstellung sowie den Verbund zur Verwendung als Arzneimittel.

Orale Dünnfilme sind dünne, meist einen pharmazeutisch aktiven Wirkstoff enthaltende Filme, die direkt in den Mundraum gelegt oder an die Mundschleimhaut angelegt werden und sich dort auflösen. Dabei handelt es sich insbesondere um dünne wirkstoffhaltige Filme auf Polymerbasis, die, wenn sie auf eine Schleimhaut, insbesondere die Mundschleimhaut, aufgebracht werden, den Wirkstoff direkt in diese abgeben. Diese oralen Dünnfilme sind in der Regel nach außen nicht klebrig. Die sehr gute Durchblutung der Mundschleimhaut sorgt für einen schnellen Übergang des Wirkstoffs in den Blutkreislauf. Dieses Darreichungssystem hat den Vorteil, dass der Wirkstoff größtenteils durch die Schleimhaut resorbiert wird und damit der "First-Pass Metabolismus", der bei der konventionellen Darreichungsform eines Wirkstoffs in Tablettenform, die in der Regel mit Flüssigkeit eingenommen werden, was nachteilig sein kann, auftritt, vermieden wird. Der Wirkstoff kann in dem Film gelöst, emulgiert oder dispergiert werden. Geeignete Wirkstoffe können nach Auflösen des oralen Dünnfilms im Mund auch abgeschluckt werden und somit über den Gastrointestinaltrakt aufgenommen werden.

Die Applikation von oralen Dünnfilmen kann problematisch sein. Durch Form, Größe, Eigenschaften und angedachtem Applikationsort des oralen Dünnfilms, aber auch durch Beeinträchtigungen des Anwenders können Schwierigkeiten auftreten, den oralen Dünnfilm wie gewünscht an der entsprechenden Stelle zu applizieren. Insbesondere bei oralen Dünnfilmen, die zur Steigerung des Wirkstofflux auf einer Oberfläche mit Mikroadeln ausgestattet sind, ist es von Bedeutung, dass diese Mikronadeln vor der Applikation nicht beschädigt werden.

Die WO 2019/101798 A1 betrifft einen mehrschichtigen oralen Wirkstoff-Film, umfassend mindestens zwei nicht-klebende Schichten, die jeweils mindestens ein hydrophiles Polymer enthalten, und mindestens eine wasserlösliche Klebeschicht, die mindestens ein wasserlösliches Polymer und mindestens einen Weichmacher enthält.

Die WO 2021/123289 A1 betrifft einen mehrschichtigen oralen Dünnfilm, umfassend eine Matrixschicht, die mindestens ein Polymer und mindestens einen pharmazeutischen Wirkstoff enthält, und mindestens eine Trägerschicht.

Die WO 2007/116959 A1 betrifft eine Mikronadelvorrichtung, die eine Vielzahl von Mikronadeln auf einem Substrat umfasst, das in der Lage ist, eine Haut zu durchstechen, wobei die Oberfläche der Mikronadeln und/oder des Substrats teilweise oder vollständig in einem festen Zustand mit einem Polyvinylalkohol enthaltenden Beschichtungsträger beschichtet ist.

Die WO 2020/169667 A1 betrifft einen Applikator mit einem Griffstuck und mit einem mit dem Griffstück verbundenen Druckkörper für ein Mikronadelpflaster sowie eine Einheit aus einem derartigen Applikator und einem Mikronadelpflaster mit einer Vielzahl von Mikronadeln.

Die WO 2004/009172 A1 betrifft eine Mikronadelvorrichtungen, die eine kegelstumpfförmige Form haben, Mikronadel-Einführvorrichtungen und Verfahren zur Verwendung von Mikronadelvorrichtungen.

Die WO 02/066016 A2 betrifft eine mucoadhäsive Arzneizubereitung zur Verabreichung von Wirkstoffen in der Veterinär- oder Humanmedizin, mit mindestens einem Wirkstoff, die dadurch gekennzeichnet ist, dass die Zubereitung eine mucoadhäsive, in wässrigen Medien zerfallsfähige Matrix aufweist.

Die WO 2019/115815 A1 betrifft ein Mikronadelarray, insbesondere ein Applikatorsystem, und deren Verwendung zur intradermalen Applikation von Wirkstoffen in Form von Salzen, insbesondere von Arzneimitteln in Form von Salzen.

Die EP 2 389 940 A2 betrifft einen Vaginalapplikator, umfassend mindestens eine erste Aufbewahrungseinheit, enthaltend mindestens ein Mucopolysaccharid, und mindestens eine zweite Aufbewahrungseinheit, enthaltend mindestens eine Säure.

Die US 2018/177991 A1 betrifft einen Mikronadelapplikator zum Einbringen von Mikronadeln in die Haut und zur Abgabe eines Arzneimittels in die Haut.

Herkömmliche Applikationshilfen können zwar Besserung verschaffen, müssen aber in der Regel mühsam vom oralen Dünnfilm gelöst werden und/oder haben einen komplizierten Aufbau. Ferner besteht die Gefahr, dass der Anwender die Applikationshilfe unbeabsichtigt verschluckt.

Der vorliegenden Anmeldung lag daher die Aufgabe zugrunde, eine Applikationshilfe für einen oralen Dünnfilm bereitzustellen, die die obigen Nachteile überwindet. Die Applikationshilfe soll eine hohe haptische Stabilität aufweisen, um den oralen Dünnfilm an dem gewünschten Ort zu applizieren. Zudem soll die Gefahr des Verschluckens der Applikationshilfe im Ganzen minimiert werden und die Gefahr der Zerstörung bzw. Beschädigung des oralen Dünnfilms durch den Anwender vor der Applikation minimiert werden.

Diese Aufgabe wurde überraschend mit einem Verbund gemäß Anspruch 1 gelöst, insbesondere mit einem Verbund, umfassend einen oralen Dünnfilm und eine Applikationshilfe für den oralen Dünnfilm, wobei der orale Dünnfilm und die Applikationshilfe so miteinander verbunden sind, dass der Anwender die Verbindung nicht trennen kann ohne den Film zu zerstören, wobei die Applikationshilfe eine Länge von 1 cm bis 10 cm, eine Breite von 0,5 cm bis 5 cm und eine Dicke von 0,1 mm bis 4 mm aufweist und die Applikationshilfe einen ersten Bereich und einen zweiten Bereich umfasst, wobei der orale Dünnfilm in dem ersten Bereich der Applikationshilfe angeordnet ist und der zweite Bereich der Applikationshilfe derart ausgestaltet ist, dass ein Anwender die Applikationshilfe so berühren kann, dass dabei der orale Dünnfilm nicht berührt wird.

Ein solcher Verbindung hat den Vorteil, dass der Anwender den oralen Dünnfilm selbst nicht berühren muss, um ihn zu applizieren. Die Berührung erfolgt vorzugsweise ausschließlich über die Applikationshilfe. Ferner kann die Applikationshilfe in einer bevorzugten Ausführungsform so ausgestaltet sein, dass sie sich selbst in der Mundhöhle des Anwenders auflöst. Durch die feste Verbindung mit der Applikationshilfe könne insbesondere sehr dünne orale Dünnfilme, insbesondere solche mit einer Dicke kleiner als 400 µm, oder solche oralen Dünnfilme, die auf einer Oberfläche mit Mikronadeln ausgestattet sind, vorteilhaft appliziert werden.

In der vorliegenden Beschreibung soll der Begriff "umfassen" auch "bestehend aus" bedeuten.

Der Begriff "Applikationshilfe" kann hier breitestmöglich verstanden werden.

Erfindungsgemäß umfasst die Applikationshilfe eine flächige Struktur mit einer Dicke von 0,1 mm bis 4 mm, vorzugsweise von 0,1 mm bis 2 mm oder von 0,5 mm bis 2mm oder von 100 µm bis 500 µm, auf deren Oberfläche der orale Dünnfilm fest angebracht ist.

Die Applikationshilfe kann dabei alle möglichen Formen aufweisen. Beispielsweise kann die Applikationshilfe im Wesentlichen rund, oval oder elliptisch ausgebildet sein.

Die Applikationshilfe kann aber auch dreieckig oder viereckig, ggf. mit abgerundeten Ecken, ausgebildet sein. Ferner sind Formen in anderen möglichen Vielecken, ggf. mit abgerundeten Ecken, möglich.

Es sind auch Mischformen möglich, wobei die Applikationshilfe beispielsweise einen ersten runden, ovalen oder elliptischen Bereich aufweist und einen daran angebrachten zweiten Bereich, der beispielsweise streifenförmig, dreieckig oder viereckig (auch vieleckig) ausgebildet ist, sodass die Applikationshilfe als Ganzes eine Form aufweist, die der Form eines Tennisschlägers ähnelt, wobei der zweite Bereich hier vorzugsweise als "Griff" und der erste Bereich zur Aufnahme des oralen Dünnfilms dient.

Unter "fest verbunden" wird vorliegend verstanden, dass die Applikationshilfe und der orale Dünnfilm so miteinander verbunden sind, dass der Anwender die Verbindung nicht trennen kann ohne den Film zu zerstören. Auch über eine längere Lagerungsdauer soll diese Verbindung möglichst nicht beeinträchtigt werden.

Der erfindungsgemäße Verbund ist erfindungsgemäß dadurch gekennzeichnet, dass die Applikationshilfe einen ersten Bereich und einen zweiten Bereich umfasst.

Dabei ist der orale Dünnfilm erfindungsgemäß in dem ersten Bereich der Applikationshilfe angeordnet.

Der zweite Bereich der Applikationshilfe ist erfindungsgemäß so ausgestaltet, dass ein Anwender die Applikationshilfe derart berühren kann, dass dabei der orale Dünnfilm nicht berührt wird.

Vorzugsweise ist der orale Dünnfilm in einem ersten Drittel oder einer ersten Hälfte der Fläche der Applikationshilfe angebracht. So bleiben zwei Drittel bzw. eine zweite Hälfte der Applikationshilfe frei und können als "Griff" zur Applikation des oralen Dünnfilms dienen.

Der erfindungsgemäße Verbund ist vorzugsweise dadurch gekennzeichnet, dass die Applikationshilfe eine Fläche aufweist, die mindestens doppelt so groß ist, vorzugsweise von 2 bis 10 mal oder von 2 bis 4 mal so groß ist, wie die Fläche des oralen Dünnfilms.

In möglichen Ausführungsformen weist die Applikationshilfe eine Fläche von 1 cm² bis 30 cm², vorzugsweise von 2 cm² bis 15 cm² oder 2 cm² bis 10 cm², auf.

In möglichen Ausführungsformen weist der orale Dünnfilm eine Fläche von 0,3 cm² bis 10 cm², vorzugsweise von 0,5 cm² bis 7 cm² auf.

Erfindungsgemäß weist die Applikationshilfe eine Länge von 1 cm bis 10 cm, vorzugsweise von 2 cm bis 5 cm, auf.

Erfindungsgemäß weist die Applikationshilfe eine Breite von 0,5 cm bis 5 cm, vorzugsweise von 1 cm bis 3 cm, auf.

In möglichen Ausführungsformen weist der orale Dünnfilm eine Länge von 0,5 cm bis 5 cm, vorzugsweise von 1 cm bis 4 cm, auf.

In möglichen Ausführungsformen weist der orale Dünnfilm eine Breite von von 0,5 cm bis 4 cm, vorzugsweise von 1 cm bis 4 cm auf.

Erfindungsgemäß weist die Applikationshilfe eine Dicke von 0,1 mm bis 4 mm, vorzugsweise von 0,1 mm bis 2 mm oder von 100 µm bis 500 µm, auf.

In möglichen Ausführungsformen weist der orale Dünnfilm eine Dicke von 0,01 mm bis 2 mm, vorzugsweise 0,05 mm bis 1 mm oder von 100 µm bis 400 µm, auf.

In möglichen Ausführungsformen weist die Applikationshilfe ein Flächengewicht von 20 g/m² bis 800 g/m², vorzugsweise von 20 g/m² bis 500 g/m², auf.

In möglichen Ausführungsformen weist der orale Dünnfilm ein Flächengewicht von 10 g/m² bis 600 g/m², vorzugsweise von 10 g/m² bis 400 g/m², auf.

Die Applikationshilfe kann einschichtig oder mehrschichtig ausgebildet sein.

Der erfindungsgemäße Verbund ist vorzugsweise dadurch gekennzeichnet, dass die Applikationshilfe mindesten ein wasserlösliches Polymer umfasst.

Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung ist, dass diese Polymere eine für die Wasserlöslichkeit ausreichende Anzahl an hydrophilen Gruppen besitzen und nicht vernetzt sind. Die hydrophilen Gruppen können nicht-ionisch, anionisch, kationisch und/oder zwitterionisch sein.

Unter wasserlöslich wird vorzugsweise eine Löslichkeit von größer als 100 g/L in Wasser bei 25°C verstanden.

Das mindestens eine wasserlösliche Polymer ist vorzugsweise ausgewählt aus der Gruppe bestehend aus Stärke und Stärkederivaten, Dextranen, Cellulosederivaten, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylaten, Polyvinylpyrrolidonen, Vinvlpyrrolidon/Vinylacetat-Copolymeren, Polyvinylalkoholen, Polyethylenoxidpolymeren, Polyacrylamiden, Polyethylenglycolen, Gelatine, Collagen, Alginaten, Pektin, Pullulan, Traganth, Chitosan, Alginsäure, Arabinogalaktan, Galaktomannan, Agar, Agarose, Carrageen und/oder natürlichen Gummen, wobei Polyvinylalkohole besonders bevorzugt sind.

Der erfindungsgemäße Verbund ist vorzugsweise dadurch gekennzeichnet, dass das mindestens eine Polymer, vorzugsweise das wasserlösliche Polymer, in einer Menge von 10 bis 100 Gew.-% oder 10 bis 95 Gew.-%, vorzugsweise von 60 bis 90 Gew.-%, besonders bevorzugt von 80 bis 90 Gew.-%, bezogen auf das Gesamtgewicht der Applikationshilfe, enthalten ist.

Der erfindungsgemäße Verbund ist vorzugsweise dadurch gekennzeichnet, dass die Applikationshilfe als Ganzes wasserlöslich ist. Unter wasserlöslich wird auch hier vorzugsweise eine Löslichkeit von größer als 100 g/L in Wasser bei 25°C verstanden.

Der erfindungsgemäße Verbund ist vorzugsweise dadurch gekennzeichnet, dass die Applikationshilfe Aromen, Geschmacksmaskierungsmittel und/oder Betäubungsmittel bzw. Lokalanaesthetika umfasst.

Als Betäubungsmittel bzw. Lokalanaesthetika sind hier beispielsweise Aminoamide, wie Lidocain, Aminoester, wie Procain, Fomocain, Quinisocain oder Cyclonin, geeignet.

Diese sind vorzugsweise in einer Menge von jeweils 0,1 bis 10 Gew.-% oder 0,1 bis 5 Gew.-%, bezogen auf das Gesamtgewicht der Applikationshilfe, enthalten.

Der erfindungsgemäße Verbund ist vorzugsweise dadurch gekennzeichnet, dass die Applikationshilfe ferner mindestens einen Hilfsstoff ausgewählt aus der Gruppe, umfassend Farbstoffe, Süßstoffe, Weichmacher, Emulgatoren, Enhancer, pH-Regulatoren, Feuchthaltemittel, Konservierungsmittel und/oder Antioxidationsmittel umfasst.

Jeder dieser Hilfsstoffe ist vorzugsweise jeweils in einer Menge von 0,1 bis 40 Gew.-%, vorzugsweise von 0,1 bis 30 Gew.-%, besonders bevorzugt von 0,1 bis 15 Gew.-%, ganz besonders bevorzugt von 0,1 bis 10 Gew.-% oder 0,1 bis 5 Gew.-%, bezogen auf das Gesamtgewicht der Applikationshilfe, enthalten.

Insbesondere über den Gehalt an Weichmacher kann die Stabilität der Applikationshilfe eingestellt werden.

Geeignete Weichmacher umfassen Glycerin.

Die bzw. der Weichmacher ist bzw. sind vorzugsweise in einer Menge von 0,1 bis 15 Gew.-%, ganz besonders bevorzugt von 0,1 bis 10 Gew.-% oder 0,1 bis 5 Gew.-%, insbesondere etwa 5 Gew.-%, bezogen auf das Gesamtgewicht der Applikationshilfe, enthalten.

Vorzugsweise ist der erfindungsgemäße Verbund dadurch gekennzeichnet, dass die Applikationshilfe in Form eines möglichst glatten Films vorliegt.

In einer anderen Ausführungsform ist der erfindungsgemäße Verbund dadurch gekennzeichnet, dass die Applikationshilfe in Form eines Hohlräume aufweisenden verfestigten Schaums vorliegt.

Durch die Hohlräume und die damit verbundene größere Oberfläche der Applikationshilfe wird insbesondere der Zutritt von Wasser bzw. Speichel oder anderen Körperflüssigkeiten in das Innere der Applikationshilfe erleichtert und somit die Auflösung der Applikationshilfe beschleunigt.

Zum anderen ist die Wandstärke der genannten Hohlräume vorzugsweise gering, da diese beispielsweise verfestigte Blasen darstellen, so dass eine schnelle Auflösung oder Zerstörung dieser Hohlräume stattfindet.

Ein weiterer Vorteil dieser Ausführungsform besteht darin, dass sich trotz des vergleichsweise hohen Flächengewichts durch die Konfektionierung als Schaum eine schnellere Trocknung realisieren lässt als bei einer vergleichbaren nicht geschäumten Zusammensetzung.

Vorzugsweise ist der erfindungsgemäße Verbund dadurch gekennzeichnet, dass die Hohlräume voneinander isoliert sind, und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium oder einem Gemisch mindestens zweier dieser Gase, gefüllt sind.

Gemäß einer anderen Ausführungsform ist vorgesehen, dass die Hohlräume miteinander in Verbindung stehen, vorzugsweise indem sie ein zusammenhängendes, die Matrix durchdringendes Kanalsystem bilden.

Vorzugsweise haben die genannten Hohlräume einen Volumenanteil von 5 bis 98 %, bevorzugt von 50 bis 80 %, bezogen auf das Gesamtvolumen der Applikationshilfe. Auf diese Weise wird die Lösung der Applikationshilfe in günstiger Weise beeinflusst.

Ferner können Applikationshilfen zur Schaumbildung oder dem erhaltenen Schaum vor oder nach dem Trocknen oberflächenaktive Stoffe bzw. Tenside hinzugefügt werden, um die Stabilität des Schaums vor oder nach dem Trocknen zu verbessern.

Ein weiterer Parameter, der die Eigenschaften der erfindungsgemäßen Applikationshilfe beeinflusst, ist der Durchmesser der Hohlräume oder Blasen. Die Blasen oder Hohlräume werden vorzugsweise mit Hilfe einer Schaumaufschlagmaschine erzeugt, mit der der Durchmesser der Blasen in einem weiten Bereich, fast beliebig, eingestellt werden kann. So kann der Durchmesser der Blasen oder Hohlräume im Bereich von 0,01 bis 350 µm liegen. Besonders bevorzugt liegt der Durchmesser im Bereich von 10 und 200 µm.

Vorzugsweise ist der erfindungsgemäße Verbund dadurch gekennzeichnet, dass der orale Dünnfilm einen oralen Dünnfilm in Form eines Films, in Form eines Schaums und/oder in Form eines Mikronadelsystems umfasst.

Der orale Dünnfilm in dem erfindungsgemäßen Verbund umfasst vorzugsweise eine Matrixschicht.

Ein Film ist vorzugsweise dadurch gekennzeichnet, dass er nicht in Form eines Schaumes vorliegt und bei optischer Analyse ohne Hilfsmittel bzw. Vergrößerung keine wesentlichen Unebenheiten aufweist. Ein Film kann beispielsweise durch Ausstreichen und Trocknen einer polymerhaltigen Lösung, Emulsion, Suspension oder Schmelze hergestellt werden.

In einer anderen Ausführungsform ist der orale Dünnfilm dadurch gekennzeichnet, dass die Matrixschicht bzw. der orale Dünnfilm in Form eines Hohlräume aufweisenden verfestigten Schaums vorliegt.

Durch die Hohlräume und die damit verbundene größere Oberfläche der Filme wird insbesondere der Zutritt von Wasser bzw. Speichel oder anderen Körperflüssigkeiten in das Innere der Darreichungsform erleichtert und somit die Auflösung der Darreichungsform und die Wirkstofffreisetzung beschleunigt.

Bei einem schnell resorbierenden Wirkstoff kann zudem durch die schnelle Auflösung die transmukosale Resorption verbessert werden.

Zum anderen ist die Wandstärke der genannten Hohlräume vorzugsweise gering, da diese beispielsweise verfestigte Blasen darstellen, so dass eine schnelle Auflösung oder Zerstörung dieser Hohlräume stattfindet.

Ein weiterer Vorteil dieser Ausführungsform besteht darin, dass sich trotz des vergleichsweise hohen Flächengewichts durch die Konfektionierung als Schaum eine schnellere Trocknung realisieren lässt als bei einer vergleichbaren nicht geschäumten Zusammensetzung.

Vorzugsweise ist der orale Dünnfilm dadurch gekennzeichnet, dass die Hohlräume voneinander isoliert sind, und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium oder einem Gemisch mindestens zweier dieser Gase, gefüllt sind.

Gemäß einer anderen Ausführungsform ist vorgesehen, dass die Hohlräume miteinander in Verbindung stehen, vorzugsweise indem sie ein zusammenhängendes, die Matrix durchdringendes Kanalsystem bilden.

Vorzugsweise haben die genannten Hohlräume einen Volumenanteil von 5 bis 98 %, bevorzugt von 50 bis 80 %, bezogen auf das Gesamtvolumen der Matrixschicht. Auf diese Weise wird der vorteilhafte Effekt, die Lösung der Matrixschicht zu beschleunigen, in günstiger Weise beeinflusst.

Ferner können der Matrix zur Schaumbildung oder dem erhaltenen Schaum vor oder nach dem Trocknen oberflächenaktive Stoffe bzw. Tenside hinzugefügt werden, um die Stabilität des Schaums vor oder nach dem Trocknen zu verbessern.

Ein weiterer Parameter, der die Eigenschaften der erfindungsgemäßen Darreichungsform beeinflusst, ist der Durchmesser der Hohlräume oder Blasen. Die Blasen oder Hohlräume werden vorzugsweise mit Hilfe einer Schaumaufschlagmaschine erzeugt, mit der der Durchmesser der Blasen in einem weiten Bereich, fast beliebig, eingestellt werden kann. So kann der Durchmesser der Blasen oder Hohlräume im Bereich von 0,01 bis 350 µm liegen. Besonders bevorzugt liegt der Durchmesser im Bereich von 10 und 200 µm.

Ein Mikronadelsystem, auch Mikronadelarray genannt, umfasst vorzugsweise ein System, umfassend eine Vielzahl an Mikronadeln an einem Träger.

Der Träger umfasst dabei vorzugsweise einen oralen Dünnfilm auf Basis mindestens eines Polymers. Die Nadeln, die vorzugsweise eine Länge von 5 µm bis 1000 µm aufweisen, können aus unterschiedlichen Materialien, wie Keramik, Stahl, Polymeren oder SiO₂ bestehen.

Dias Mikronadelsystem umfasst vorzugsweise Mikronadeln mit einer Länge von 100 µm bis 600 µm, vorzugsweise von 250 µm bis 350 µm, besonders bevorzugt von etwa 300 µm.

Der erfindungsgemäße Verbund ist vorzugsweise dadurch gekennzeichnet, dass das Mikronadelsystem ein Mikronadelsystem auf Basis von Glas, SiO₂, Stahl, Keramik, Stärke und Stärkederivaten, Dextranen, Cellulosederivaten, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylaten, Polyvinylpyrrolidonen, Polyvinylalkoholen, Poly(lactid-co-glycoid), Hyaluronsäure, Polyethylenoxidpolymeren, Polyacrylamiden, Polyethylenglycolen, Gelatine, Collagen, Alginaten, Pektin, Pullulan, Traganth, Chitosan, Alginsäure, Arabinogalaktan, Galaktomannan, Agar, Agarose, Carrageen und natürlichen Gummen, ist, wobei diese Gruppe nicht abschließend ist.

Die Mikronadeln werden vorzugsweise mit einer Klebeschicht auf dem oralen Dünnfilm befestigt. Als Klebeschicht eignet sich beispielsweise eine Klebeschicht, wie diese für die Verbindung der Applikationshilfe mit dem oralen Dünnfilm definiert ist.

Alternativ kann der orale Dünnfilm mit Mikronadeln auch dadurch hergestellt werden, dass bei der Herstellung des oralen Dünnfilms eine Negativform der Mikronadeln eingesetzt wird, sodass der orale Dünnfilm und die Mikronadeln monolithisch vorliegen.

Alternativ können die Mikronadeln mit allen geeigneten Befestigungsmitteln auf dem oralen Dünnfilm befestigt werden.

Vorzugsweise umfassen die Nadeln ein Polymer, das auch in dem Träger vorhanden ist.

Der erfindungsgemäße Verbund ist vorzugsweise dadurch gekennzeichnet, dass bei dem Vorhandensein eines Mikronadelsystems, insbesondere angrenzend zu den Mikronadeln, eine Erhöhung vorhanden ist, welche vorzugsweise auf der Applikationshilfe befestigt ist.

Die Erhöhung ist dabei vorzugsweise so ausgestaltet, dass dadurch die Mikronadeln geschützt, insbesondere dadurch, dass die Erhöhung höher als die Mikronadeln ausgebildet ist, dass die Erhöhung vor den Mikronadeln berührt wird.

Die Erhöhung ist ferner vorzugsweise dadurch gekennzeichnet, dass die umschlossene Fläche mindestens die selbe Fläche wie das Mikronadelsystem, besonders bevorzugt die Fläche des oralen Dünnfilms, umfasst und die Höhe der Erhöhung mindestens der Höhe der Mikronadeln entspricht.

Dadurch kann sichergestellt werden, dass die Mikronadeln von allen Seiten durch die Erhöhung geschützt sind.

Vorzugsweise ist die besagte Erhöhung in Form eines durchgängigen Formkörpers ausgeformt und umrahmt eine quadratisch, rechteckig, elliptisch oder kreisförmig Grundfläche, wobei diese insbesondere an der Anordnung des Mikronadelsystems angepasst ist.

In einer anderen Ausführungsform ist die Erhöhung kein durchgängiger Formkörper, sondern rahmt das Mikronadelsystems partiell ein.

Die Erhöhung ist vorzugsweise dadurch gekennzeichnet, dass die Erhöhung ein wasserlösliches Polymer (wie oben definiert) umfasst.

Vorzugsweise umfasst die Erhöhung dasselbe Material wie die Applikationshilfe.

Unabhängig von der Form des oralen Dünnfilms, ist der orale Dünnfilm vorzugsweise dadurch gekennzeichnet, dass der orale Dünnfilm mindestens ein wasserlösliches Polymer (wie oben definiert) umfasst.

Das mindestens eine wasserlösliche Polymer ist vorzugsweise ausgewählt aus der Gruppe bestehend aus Stärke und Stärkederivaten, Dextranen, Cellulosederivaten, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylaten, Polyvinylpyrrolidonen, Vinvlpyrrolidon/Vinylacetat-Copolymeren, Polyvinylalkoholen, Polyethylenoxidpolymeren, Polyacrylamiden, Polyethylenglycolen, Gelatine, Collagen, Alginaten, Pektin, Pullulan, Traganth, Chitosan, Alginsäure, Arabinogalaktan, Galaktomannan, Agar, Agarose, Carrageen und/oder natürlichen Gummen, wobei Polyvinylalkohole besonders bevorzugt sind.

Der orale Dünnfilm ist vorzugsweise dadurch gekennzeichnet, dass das mindestens eine Polymer, vorzugsweise das wasserlösliche Polymer, in einer Menge von 10 bis 100 Gew.-% oder von 10 bis 99 Gew.-% oder von 30 bis 95 Gew.-%, vorzugsweise von 60 bis 90 Gew.-%, besonders bevorzugt von 70 bis 90 Gew.-%, bezogen auf das Gesamtgewicht der Matrixschicht, in dieser vorliegt.

Der erfindungsgemäße Verbund ist vorzugsweise dadurch gekennzeichnet, dass der orale Dünnfilm als Ganzes wasserlöslich ist. Unter wasserlöslich wird auch hier vorzugsweise eine Löslichkeit von größer als 100 g/L in Wasser bei 25°C verstanden.

Der erfindungsgemäße Verbund ist vorzugsweise dadurch gekennzeichnet, dass der orale Dünnfilm mindestens einen pharmazeutisch aktiven Wirkstoff umfasst.

Der mindestens eine pharmazeutisch aktive Wirkstoff in dem oralen Dünnfilm ist vorzugsweise aus der Gruppe, bestehend aus Hypnotica, Sedativa, Antieleptica, Weckaminen, Psychoneurotropica, Neuroleptika, Neuro-Muskelblockern, Antispasmodica, Antihistaminica, Antiallergica, Cardiotonica, Antiarrhythmica, Diuretica, Hypotensiva, Vasopressoren, Antitussiva, Expectorantia, Analgetica, Thyroidhormonen, Sexualhormonen, Glucocorticoidhormonen, Antidiabetica, Antitumor-Wirkstoffen, Antibiotica, Chemotherapeutica, Narcotica, Anti-Parkinson-Wirkstoffen, Antialzheimer-Wirkstoffen und/oder Triptanen ausgewählt, wobei diese Gruppe nicht abschließend ist.

Spezifische Beispiele umfassen Acetaminophen, Adrenalin, Alprazolam, Amlodipin, Anastrozol, Apomorphin, Aripiprazol, Atorvastatin, Baclofen, Benzocain, Benzocain/Menthol, Benzydamin, Buprenorphin, Buprenorphin/Naloxon, Buprenorphin/Naloxon/Cetirizin, Cetirizin, Chlorpheniramin, Clomipramin, Dexamethason, Dextromethorphan, Dextromethorphan/Phenylephrin, Diclofenac, Diphenhydramin, Diphenhydramin/Phenylephrin, Donepezil Dronabinol, Epinephrin, Escitalopram, Famotidin, Fentanyl, Glimepirid, GLP-1 Peptiden, Granisetron, Insulin, Insulin Nanopartikel, Insulin/GLP-1 Nanopartikel, Ketamin, Ketoprofen, Ketotifen, Koffein, Levocetirizin, Loperamid, Loratadin, Meclizin, Methylphenidat, Midazolam, Mirodenafil, Montelukast, Multimeric-001, Naloxon, Nikotin, Nitroglycerin, Olanzapin, Olopatadin, Ondansetron, Oxybutynin, Pektin, Pektin/Menthol, Pectin/Ascorbinsäure, PediaSUNAT (Artesunat und Amodiaquin), Piroxicam, Phenylephrin, Prednisolon, Pseudoephedrin, Risperidon, Rivastigmin, Rizatriptan, Selegiline, Senna Glykosiden, Sildenafil Zitrat, Simethicon, Sumatriptan, Tadalafil, Testosteron, Triamcinolon Azetonid, Triptan, Tropicamide, Voglibose, Zolmitriptan, Zolpidem, oder pharmazeutisch akzeptable Salze dieser Verbindungen.

Der pharmazeutische Wirkstoff kann auch eine Mischung von unterschiedlichen Wirkstoffen sein.

Die Menge von Wirkstoff in dem oralen Dünnfilm hängt von dessen Art ab und beträgt üblicherweise 0,01 bis 70 Gew.-%, bevorzugt 0,1 bis 50 Gew.-%, besonders bevorzugt 1 bis 40 Gew.-%, bezogen auf das Gesamtgewicht des oralen Dünnfilms.

Es kann von Vorteil sein, dass der orale Dünnfilm außerdem noch mindestens einen Hilfsstoff, ausgewählt aus der Gruppe, umfassend Farbstoffe, Aromastoffe, Süßstoffe, geschmacksmaskierende Mittel, Tenside, Enhancer, pH-Regulatoren, Konservierungsmittel, Antioxidationsmittel und/oder Weichmacher, enthält.

Diese Hilfsstoffe sind vorzugsweise jeweils in einer Menge von 0,001 bis 20 Gew.-% in dem oralen Dünnfilm enthalten.

Der orale Dünnfilm ist prinzipiell in der Anzahl der enthaltenen Schichten nicht beschränkt.

So sind Ausführungsformen denkbar, bei denen der orale Dünnfilm weitere Schichten umfasst. Die obigen Definitionen gelten analog für die weiteren Schichten.

Der erfindungsgemäße Verbund ist vorzugsweise dadurch gekennzeichnet, dass die Applikationshilfe und der orale Dünnfilm mittels einer Klebeschicht, vorzugsweise einer wasserlöslichen Klebeschicht, mittels einer Siegelung, vorzugsweise einer Heißsiegelung miteinander und/oder mittels Aufeinanderbeschichtung miteinander verbunden sind.

Unter Klebeschicht wird eine Schicht verstanden, die als Klebstoff, wie in der DIN EN 923:2016-03 definiert, wirken kann.

Als Klebeschichten eignen sich insbesondere wasserlösliche Klebeschichten, wie diese in der DE 10 2014 127 452 A1 beschrieben werden, deren diesbezüglicher Inhalt hiermit vollständig einbezogen ist.

Geeignete Klebeschichten umfassen mindestens ein wasserlösliches Polymer und mindestens einen Weichmacher, wobei das mindestens eine wasserlösliche Polymer in der mindestens einen wasserlöslichen Klebeschicht Schellack, ein Vinylpyrrolidon-/Vinylacetat-Copolymer, ein Polyvinylcaprolactam-/Polyvinylacetat-/Polyethylenglycol-Copolymer, Hydroxypropylcellulose oder Hydroxypropylmethylcellulose und/oder Polyvinylpyrrolidon umfasst, und wobei der mindestens eine Weichmacher in der mindestens einen wasserlöslichen Klebeschicht Glycerin, Polyethylenglycol, insbesondere Polyethylenglycol 200, und/oder Tributylcitrat umfasst. Das Gewichtsverhältnis des mindestens einen wasserlöslichen Polymers zu dem mindestens einen Weichmacher in der mindestens einen Klebeschicht beträgt vorzugsweise etwa 85 bis 50 zu etwa 15 bis 50.

Eine derartige Klebeschicht, die mindestens ein wasserlösliches Polymer und mindestens einen Weichmacher enthält, kann als dazwischenliegende wasserlösliche Klebeschicht zwei weitere Schichten, die an sich nicht klebrig sind, fest miteinander verkleben und somit den Aufbau von mehrschichtigen Verbunden ermöglichen.

Unter Siegelung wird jede mögliche Art und Weise verstanden durch die die Applikationshilfe und der oralen Dünnfilm miteinander verbunden werden können. Dies umfasst beispielsweise aber nicht abschließend Verbindungen durch Prägung und/oder Heißsiegelung.

Unter Heißsiegelung wird eine Verbindung der Applikationshilfe und des oralen Dünnfilms durch eine punktuelle Erwärmung und Anpressung verstanden. Durch die punktuelle Erwärmung schmilzt das mindestens eine Polymer, das jeweils in der Applikationshilfe bzw. in dem oralen Dünnfilms vorhanden ist, was nach dem erneuten Erkalten zu einer festen Verbindung führt. Bevorzugt wird dabei punktuell auf eine Temperatur erwärmt, die oberhalb der Schmelztemperatur bzw. Glasübergangstemperatur des jeweiligen Polymers liegt.

Übliche Temperaturen für eine Heißsiegelung betragen etwa 50°C bis 200°C.

Die Heißsiegelung wird bevorzugt für etwa 5 Sekunden, bevorzugt etwa 3 Sekunden und besonders bevorzugt etwa 2 Sekunden oder weniger bei einer Temperatur von etwa 50°C bis 200°C durchgeführt.

Unter Aufeinanderbeschichtung versteht man vorzugsweise das direkte Aufbringen einer zweiten Schicht auf eine bereits vorhanden erste Schicht.

So kann beispielsweise die Applikationshilfe vorgelegt werden und es wird die Zusammensetzung des oralen Dünnfilms in einer verarbeitbaren Form, beispielsweise als Lösung, Emulsion oder Suspension auf die Applikationshilfe aufgetragen (beschichtet). Nach dem Trocknen entsteht so ein fester Verbund. Es kann auch der orale Dünnfilm vorgelegt werden und mit der Applikationshilfe beschichtet werden.

Der erfindungsgemäße Verbund ist ferner vorzugsweise dadurch gekennzeichnet, dass sich die Applikationshilfe innerhalb von 20 s bis 5 min, vorzugsweise von 30 s bis 3 min, in der Mundhöhle eines Patienten auflöst.

Der erfindungsgemäße Verbund ist ferner vorzugsweise dadurch gekennzeichnet, dass sich die Applikationshilfe und der orale Dünnfilm innerhalb von 20 s bis 5 min, vorzugsweise von 30 s bis 2 min, in der Mundhöhle eines Patienten auflöst.

Die vorliegende Erfindung betrifft auch ein Verfahren zur Herstellung des Verbundes, vorzugsweise wie vorstehend definiert, umfassend die Schritte:
- Bereitstellen einer Applikationshilfe, welche eine Länge von 1 cm bis 10 cm, eine breite von 0,5 bis 5 cm und eine Dicke von 0,1 mm bis 4 mm aufweist
- Bereitstellen eines oralen Dünnfilms, und
- festes Verbinden der Applikationshilfe mit dem oralen Dünnfilm, wobei der orale Dünnfilm in dem ersten Bereich der Applikationshilfe angeordnet ist und der zweite Bereich der Applikationshilfe derart ausgestaltet ist, dass ein Anwender die Applikationshilf so berühren kann, dass dabei der orale Dünnfilm nicht berührt wird.

Alle vorstehen für den Verbund, den oralen Dünnfilm und die Applikationshilfe definierten Ausführungsformen gelten analog für das erfindungsgemäße Verfahren.

Es ist dabei unerheblich, ob in einem ersten Schritt die Applikationshilfe oder der orale Dünnfilm bereitgestellt wird.

Vorzugsweise wird die Applikationshilfe und/oder der orale Dünnfilm durch ein Verfahren bereitgestellt, dass das Herstellen einer Lösung, Emulsion, Schmelze oder Suspension, umfassend alle Inhaltsstoffe der Applikationshilfe bzw. des oralen Dünnfilms, gefolgt von dem Ausstreichen und Trocknen der Lösung, Emulsion, Schmelze oder Suspension.

Aus diesen getrockneten Lösungen, Emulsionen, Schmelzen oder Suspensionen können alle geeigneten Formen ausgeschnitten oder gestanzt werden.

Falls Mikronadeln vorhanden sein sollen, werden diese vorzugsweise mit einer Klebeschicht auf dem oralen Dünnfilm befestigt. Als Klebeschicht eignet sich hier beispielsweise eine Klebeschicht, wie diese oben für die Verbindung der Applikationshilfe mit dem oralen Dünnfilm definiert ist.

Alternativ kann der orale Dünnfilm mit Mikronadeln auch dadurch hergestellt werden, dass bei der Herstellung des oralen Dünnfilms eine Negativform der Mikronadeln eingesetzt wird, sodass der orale Dünnfilm und die Mikronadeln monolithisch vorliegen.

Alternativ können die Mikronadeln mit allen geeigneten Befestigungsmitteln auf dem oralen Dünnfilm befestigt werden.

Wenn die Applikationshilfe und/oder der orale Dünnfilm in Form eines Schaumes vorliegt, umfasst das Verfahren zudem den Schritt des Aufschäumens der Lösung, Emulsion, Schmelze oder Suspension durch Eintragen eines Gases oder Gasgemisches, durch chemische Gaserzeugung oder durch Entspannen eines gelösten Gases.

Das feste Verbinden erfolgt vorzugsweise mittels einer wasserlöslichen Klebeschicht, mittels einer Siegelung, vorzugsweise einer Heißsiegelung und/oder mittels Aufeinanderbeschichtung, wie vorstehend definiert.

Zudem beschrieben wird ein Verbund erhältlich nach dem vorstehend beschriebenen Verfahren.

Alle vorstehend für den Verbund, den oralen Dünnfilm und die Applikationshilfe definierten Ausführungsformen gelten analog für einen Verbund erhältlich nach dem vorstehend beschriebenen Verfahren.

Außerdem betrifft die vorliegende Erfindung einen Verbund, wie vorstehend beschrieben als Arzneimittel.

Alle vorstehen für den Verbund, den oralen Dünnfilm und die Applikationshilfe definierten Ausführungsformen gelten analog für die erfindungsgemäße medizinische Verwendung.

Beschreibung der Figuren:

### Figur 1:

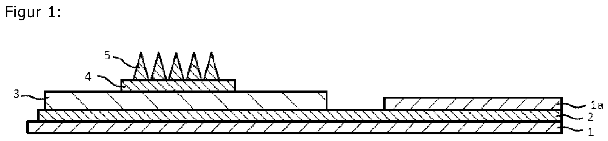

Die Figur 1 zeigt eine mögliche Ausgestaltung des Verbunds, umfassend einen oralen Dünnfilm und eine Applikationshilfe für den oralen Dünnfilm. Der Verbund weist dabei einen ersten Bereich der Applikationshilfe auf, auf dem der orale Dünnfilm angeordnet ist und einen zweiten Bereich der Applikationshilfe, der so ausgestaltet ist, dass ein Anwender die Applikationshilfe so berühren kann, dass dabei der orale Dünnfilm nicht berührt wird.

Auf eine Applikationshilfe (1) wird eine Klebeschicht (2) aufgetragen, auf die der orale Dünnfilm (3) in einem ersten Bereich aufgebracht wird. Damit der Benutzer die Klebeschicht nicht berühren muss, kann auf der Klebeschicht optional noch einmal ein Stück der Applikationshilfe (1a) in einem zweiten Bereich aufgetragen werden.

Der orale Dünnfilm kann optional mit Mikronadeln (5) ausgestattet werden. Diese Mikronadeln (5) können entweder mit einer weiteren Klebeschicht (4) auf dem oralen Dünnfilm aufgebracht werden oder mittels alternativer Befestigungsmaßnahmen dort befestigt werden.

### Figur 2:

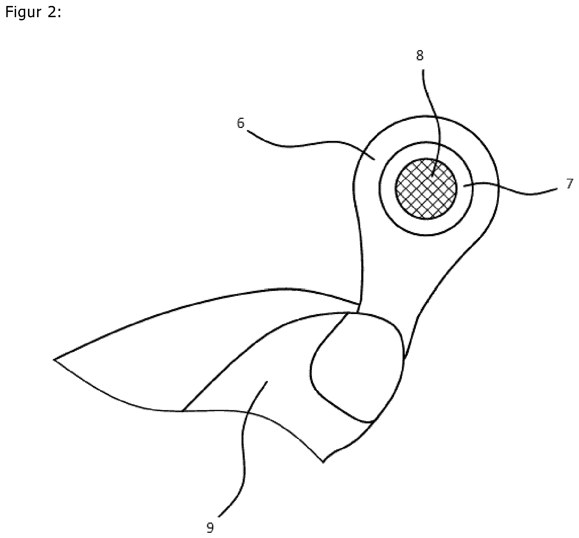

Die Figur 2 zeigt weitere eine mögliche Ausgestaltung des Verbunds, umfassend einen oralen Dünnfilm und eine Applikationshilfe für den oralen Dünnfilm. Der Verbund weist einen ersten Bereich der Applikationshilfe auf, auf dem der orale Dünnfilm angeordnet ist, und einen zweiten Bereich der Applikationshilfe, der so ausgestaltet ist, dass ein Anwender die Applikationshilfe so berühren kann, dass dabei der orale Dünnfilm nicht berührt wird. Der Anwender wird hierbei über die stilisierte Hand (9) dargestellt.

Auf eine Applikationshilfe (6) wird ein oraler Dünnfilm (7) in einem Bereich aufgebracht, der sich von dem Bereich unterscheidet, den der Benutzer mit seiner Hand (9) berührt. Der orale Dünnfilm (7) kann optional mit Mikronadeln (8) ausgestattet sein.

### Figur 3:

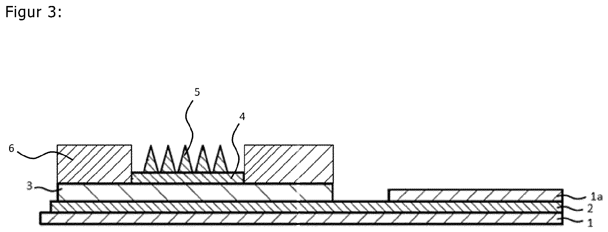

Die Figur 3 zeigt den Querschnitt einer möglichen Ausgestaltung des Verbunds, umfassend einen oralen Dünnfilm und eine Applikationshilfe für den oralen Dünnfilm. Der Verbund weist einen ersten Bereich der Applikationshilfe auf, auf dem der orale Dünnfilm, die Mikronadeln und eine die Mikronadeln einrahmende Erhöhung angeordnet sind, um sicherzustellen, dass die Mikronadeln nicht beschädigt werden.

Auf eine Applikationshilfe (1) wird eine Klebeschicht (2) aufgetragen, auf die der orale Dünnfilm (3) in einem ersten Bereich aufgebracht wird. Damit der Benutzer die Klebeschicht nicht berühren muss, kann auf der Klebeschicht optional noch einmal ein Stück der Applikationshilfe (1a) in einem zweiten Bereich aufgetragen werden.

Der orale Dünnfilm ist mit Mikronadeln (5) ausgestattet, wobei eine Erhöhung (6) auf der Applikationshilfe (1) angebracht ist, welche die Mikronadeln (5) einrahmt. Die Mikronadeln (5) und die Erhöhung (6) können entweder mit einer weiteren Klebeschicht (4) auf dem oralen Dünnfilm aufgebracht werden oder mittels alternativer Befestigungsmaßnahmen dort befestigt werden.

### Figur 4:

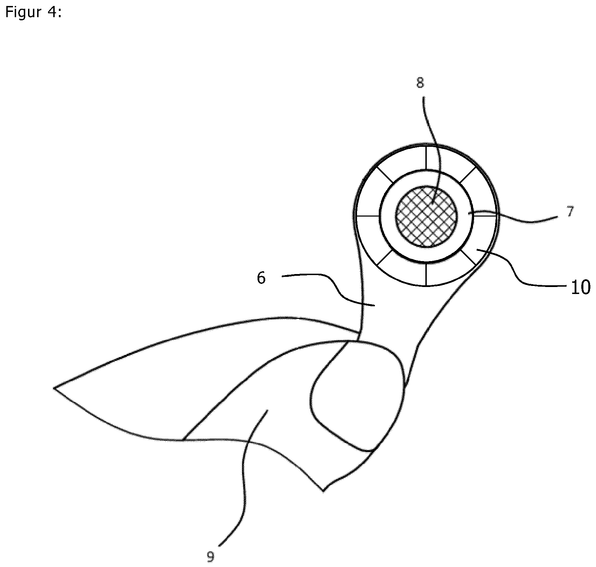

Die Figur 4 zeigt weitere eine mögliche Ausgestaltung des Verbunds, umfassend einen oralen Dünnfilm und eine Applikationshilfe für den oralen Dünnfilm. Der Verbund weist einen ersten Bereich der Applikationshilfe auf, auf dem der orale Dünnfilm, die Mikronadeln und eine die Mikronadeln einrahmende Erhöhung angeordnet sind, um sicherzustellen, dass die Mikronadeln nicht beschädigt werden. Der zweite Bereich der Applikationshilfe ist so ausgestaltet, dass ein Anwender die Applikationshilfe so berühren kann, dass dabei der orale Dünnfilm nicht berührt wird. Der Anwender wird hierbei über die stilisierte Hand (9) dargestellt.

Auf eine Applikationshilfe (6) wird ein oraler Dünnfilm (7) in einem Bereich aufgebracht, der sich von dem Bereich unterscheidet, den der Benutzer mit seiner Hand (9) berührt. Der orale Dünnfilm (7) ist mit Mikronadeln (8) ausgestattet, wobei eine Erhöhung (10) auf der Applikationshilfe (6) angebracht ist, welche die Mikronadeln (8) einrahmt.

Die Erfindung wird nachfolgend anhand von nicht beschränkenden Beispielen näher erläutert.

### Beispiele

Ein oraler Dünnfilm, in diesem Fall ein Mehrschichtsystem mit Mikronadeln, wurde mit einem Polyvinylalkoholschaum als Applikationshilfe versehen.

Der orale Dünnfilm wurde mittels einer Klebeschicht auf die Applikationshilfe aufgebracht.

Die Applikationshilfe weist die folgende Rezeptur auf.

| Material | Anteil % (trocken) | Beschreibung |
|---|---|---|
| Polyvinylalkohol 4-88 | 84,8 | Polymer |
| FD&C-Rot no.40 | 0,2 | Farbstoff |
| Saccharin Na | 1,0 | Süßungsmittel |
| Sucralose | 2,0 | Süßungsmittel |
| Glycerin | 5,0 | Weichmacher |
| Cherry Aroma | 6,0 | Geschmacksstoff |
| Menthol | 1,0 | Geschmacksstoff |

Es wird ein stabiler Verbund erhalten. Der sehr dünne orale Dünnfilm, der zum Falten neigt, konnte appliziert werden. Insbesondere durch die Anwesenheit der Mikronadeln ist eine sachgemäße Applikation nötig, da eine unsachgemäße Applikation zum Abbrechen der Mikronadeln führt.

## Patentansprüche

1. Verbund, umfassend einen oralen Dünnfilm und eine Applikationshilfe für den oralen Dünnfilm, **dadurch gekennzeichnet, dass** der orale Dünnfilm und die Applikationshilfe so miteinander verbunden sind, dass der Anwender die Verbindung nicht trennen kann ohne den Film zu zerstören, wobei die Applikationshilfe eine Länge von 1 cm bis 10 cm, eine Breite von 0,5 cm bis 5 cm und eine Dicke von 0,1 mm bis 4 mm aufweist und die Applikationshilfe einen ersten Bereich und einen zweiten Bereich umfasst, wobei der orale Dünnfilm in dem ersten Bereich der Applikationshilfe angeordnet ist und der zweite Bereich der Applikationshilfe derart ausgestaltet ist, dass ein Anwender die Applikationshilfe so berühren kann, dass dabei der orale Dünnfilm nicht berührt wird.

2. Verbund gemäß irgendeinem der vorhergehenden Ansprüche, **dadurch gekennzeichnet, dass** die Applikationshilfe eine Fläche aufweist, die mindestens doppelt so groß ist, vorzugsweise von 2 bis 10 mal so groß ist, wie die Fläche des oralen Dünnfilms.

3. Verbund gemäß irgendeinem der vorhergehenden Ansprüche, **dadurch gekennzeichnet, dass** die Applikationshilfe eine Fläche von 1 cm² bis 30 cm² aufweist.

4. Verbund gemäß irgendeinem der vorhergehenden Ansprüche, **dadurch gekennzeichnet, dass** die Applikationshilfe mindestens ein wasserlösliches Polymer umfasst.

5. Verbund gemäß irgendeinem der vorhergehenden Ansprüche, **dadurch gekennzeichnet, dass** die Applikationshilfe als Ganzes wasserlöslich ist.

6. Verbund gemäß irgendeinem der vorhergehenden Ansprüche, **dadurch gekennzeichnet, dass** die Applikationshilfe Aromen, Geschmacksmaskierungsmittel und/oder Betäubungsmittel umfasst.

7. Verbund gemäß irgendeinem der vorangehenden Ansprüche, **dadurch gekennzeichnet, dass** der orale Dünnfilm einen oralen Dünnfilm in Form eines Films, in Form eines Schaums und/oder in Form eines Mikronadelsystems umfasst.

8. Verbund gemäß irgendeinem der vorhergehenden Ansprüche, **dadurch gekennzeichnet, dass** der orale Dünnfilm mindestens ein wasserlösliches Polymer umfasst.

9. Verbund gemäß irgendeinem der vorhergehenden Ansprüche, **dadurch gekennzeichnet, dass** der orale Dünnfilm mindestens einen pharmazeutisch aktiven Wirkstoff umfasst.

10. Verbund gemäß irgendeinem der vorhergehenden Ansprüche, **dadurch gekennzeichnet, dass** die Applikationshilfe und der orale Dünnfilm mittels einer Klebeschicht, vorzugsweise einer wasserlöslichen Klebeschicht, mittels einer Siegelung, vorzugsweise einer Heißsiegelung, miteinander und/oder mittels Aufeinanderbeschichtung miteinander verbunden sind.

11. Verbund gemäß irgendeinem der vorhergehenden Ansprüche, **dadurch gekennzeichnet, dass** sich die Applikationshilfe innerhalb von 20 s bis 5 min in der Mundhöhle eines Patienten auflöst.

12. Verfahren zur Herstellung eines Verbundes gemäß irgendeinem der Anspruch 1 bis 11, umfassend die Schritte:
- Bereitstellen einer Applikationshilfe, welche eine Länge von 1 cm bis 10 cm, eine Breite von 0,5 cm bis 5 cm und eine Dicke von 0,1 mm bis 4 mm aufweist,
- Bereitstellen eines oralen Dünnfilms, und
- festes Verbinden der Applikationshilfe mit dem oralen Dünnfilm,
wobei der orale Dünnfilm in dem ersten Bereich der Applikationshilfe angeordnet ist und der zweite Bereich der Applikationshilfe derart ausgestaltet ist, dass ein Anwender die Applikationshilfe so berühren kann, dass dabei der orale Dünnfilm nicht berührt wird.

13. Verfahren gemäß Anspruch 12, **dadurch gekennzeichnet, dass** das feste Verbinden mittels einer Klebeschicht, vorzugsweise einer wasserlöslichen Klebeschicht, mittels einer Siegelung, vorzugsweise einer Heißsiegelung, und/oder mittels Aufeinanderbeschichtung erfolgt.

14. Verbund gemäß irgendeinem der Ansprüche 1 bis 11 zur Verwendung als Arzneimittel.

## Claims

1. A composite comprising an oral thin film and an application aid for the oral thin film, **characterized in that** the oral thin film and the application aid are firmly joined to each other, wherein the application aid and the oral thin film are joined to each other in such a way that the user cannot separate the joint without destroying the film, wherein the application aid has a length of 1 cm to 10 cm, a width of 0.5 cm to 5 cm, and a thickness of 0.1 mm to 4 mm, and the application aid comprises a first area and a second area, wherein the oral thin film is disposed in the first area of the application aid, and the second area of the application aid is designed in such a way that a user can touch the application aid in such a way that the oral thin film is not touched.

2. The composite according to any one of the preceding claims, **characterized in that** the application aid has a surface area which is at least twice as large, preferably 2 to 10 times as large, as the surface area of the oral thin film.

3. The composite according to any one of the preceding claims, **characterized in that** the application aid has a surface area of 1 cm² to 30 cm².

4. The composite according to any one of the preceding claims, **characterized in that** the application aid comprises at least one water-soluble polymer.

5. The composite according to any one of the preceding claims, **characterized in that** the application aid as a whole is water-soluble.

6. The composite according to any one of the preceding claims, **characterized in that** the application aid comprises flavors, flavor masking agents and/or anesthetics.

7. The composite according to any one of the preceding claims, **characterized in that** the oral thin film comprises an oral thin film in the form of a film, in the form of a foam, and/or in the form of a microneedle system.

8. The composite according to any one of the preceding claims, **characterized in that** the oral thin film comprises at least one water-soluble polymer.

9. The composite according to any one of the preceding claims, **characterized in that** the oral thin film comprises at least one active pharmaceutical ingredient.

10. The composite according to any one of the preceding claims, **characterized in that** the application aid and the oral thin film are joined to each other by means of an adhesive layer, preferably a water-soluble adhesive layer, by means of a seal, preferably a heat seal, and/or are joined to each other by means of coating one on top of the other.

11. The composite according to any one of the preceding claims, **characterized in that** the application aid dissolves in the oral cavity of a patient within 20 s to 5 min.

12. A method for preparing a composite according to any one of the claims 1 to 11, comprising the steps of:
- providing an application aid, having a length of 1 cm to 10 cm, a width of 0.5 cm to 5 cm, and a thickness of 0.1 mm to 4 mm,
- providing an oral thin film, and
- firmly joining the application aid to the oral thin film, wherein the oral thin film is disposed in the first area of the application aid and the second area of the application aid is designed in such a way that a user can touch the application aid in such a way that the oral thin film is not touched.

13. The method according to claim 12, **characterized in that** the firm joining is carried out by means of an adhesive layer, preferably a water-soluble adhesive layer, by means of a seal, preferably a heat seal, and/or by means of coating one on top of the other.

14. A composite according to any one of the claims 1 to 11 for use as a medicinal product.

## Revendications

1. Composite, comprenant un film mince oral et un auxiliaire d'application pour le film mince oral, **caractérisé en ce que** le film mince oral et l'auxiliaire d'application sont reliés ensemble de sorte que l'utilisateur ne puisse pas rompre la liaison sans détruire le film, dans lequel l'auxiliaire d'application présente une longueur de 1 cm à 10 cm, une largeur de 0,5 cm à 5 cm et une épaisseur de 0,1 mm à 4 mm, et l'auxiliaire d'application comprend une première zone et une deuxième zone, dans lequel le film mince oral est disposé dans la première zone de l'auxiliaire d'application et la deuxième zone de l'auxiliaire d'application est conçue de sorte qu'un utilisateur puisse toucher l'auxiliaire d'application de telle manière que le film mince oral ne soit ce faisant pas touché.

2. Composite selon l'une quelconque des revendications précédentes, **caractérisé en ce que** l'auxiliaire d'application présente une surface qui est au moins deux fois plus grande, de préférence de 2 à 10 fois plus grande que la surface du film mince oral.

3. Composite selon l'une quelconque des revendications précédentes, **caractérisé en ce que** l'auxiliaire d'application présente une surface de 1 cm² à 30 cm².

4. Composite selon l'une quelconque des revendications précédentes, **caractérisé en ce que** l'auxiliaire d'application comprend au moins un polymère hydrosoluble.

5. Composite selon l'une quelconque des revendications précédentes, **caractérisé en ce que** l'auxiliaire d'application dans son ensemble est hydrosoluble.

6. Composite selon l'une quelconque des revendications précédentes, **caractérisé en ce que** l'auxiliaire d'application comprend des arômes, des agents de masquage du goût et/ou des anesthésiques.

7. Composite selon l'une quelconque des revendications précédentes, **caractérisé en ce que** le film mince oral comprend un film mince sous forme d'un film, sous forme d'une mousse et/ou sous forme d'un système de microaiguilles.

8. Composite selon l'une quelconque des revendications précédentes, **caractérisé en ce que** le film mince oral comprend au moins un polymère hydrosoluble.

9. Composite selon l'une quelconque des revendications précédentes, **caractérisé en ce que** le film mince oral comprend au moins un principe actif pharmaceutique.

10. Composite selon l'une quelconque des revendications précédentes, **caractérisé en ce que** l'auxiliaire d'application et le film mince oral sont reliés entre eux au moyen d'une couche adhésive, de préférence d'une couche adhésive hydrosoluble, au moyen d'un scellement, de préférence un scellement à chaud, et/ou au moyen d'un revêtement superposé.

11. Composite selon l'une quelconque des revendications précédentes, **caractérisé en ce que** l'auxiliaire d'application se dissout dans la cavité buccale d'un patient en l'espace de 20 s à 5 min.

12. Procédé de fabrication d'un composite selon l'une quelconque des revendications 1 à 11, comprenant les étapes :
- de fourniture d'un auxiliaire d'application, lequel présente une longueur de 1 cm à 10 cm, une largeur de 0,5 cm à 5 cm et une épaisseur de 0,1 mm à 4 mm,
- de fourniture d'un film mince oral, et
- de liaison solide de l'auxiliaire d'application au film mince oral,
dans lequel le film mince oral est disposé dans la première zone de l'auxiliaire d'application et la deuxième zone de l'auxiliaire d'application est conçue de sorte qu'un utilisateur peut toucher l'auxiliaire d'application de telle manière que le film mince oral ne soit ce faisant pas touché.

13. Procédé selon la revendication 12, **caractérisé en ce que** la liaison solide s'effectue au moyen d'une couche adhésive, de préférence d'une couche adhésive hydrosoluble, au moyen d'un scellement, de préférence d'un scellement à chaud, et/ou au moyen d'un revêtement superposé.

14. Composite selon l'une quelconque des revendications 1 à 11 pour son utilisation comme médicament.
